# FASCICULE DE BREVET EUROPEEN

(11) **EP 2 762 170 B1**
(45) Date de publication et mention de la délivrance du brevet: **09.12.2015**
(21) Numéro de dépôt: 14153402.4
(22) Date de dépôt: 31.01.2014
(51) Int. Cl.: A61L 9/20, B01D 53/00, B01D 53/32, B01D 53/86

(54) **Dispositif, système et procédé de traitement de gaz par la méthode décharge couche diélectrique**
Vorrichtung, System und Verfahren zur Gasbehandlung mit dielektrischer Sperrschichtentladung
Apparatus, system and process for gas treatment with dielectrical barrier discharge

(30) Priorité: 01.02.2013 FR 1350906
(43) Date de publication de la demande: 06.08.2014
(73) Titulaire: Compagnie Industrielle D'Applications Thermiques, 01350 Culoz (FR); Ecole Nationale Supérieure de Chimie de Rennes, 35708 Rennes Cedex 7 (FR); Centre National de la Recherche Scientifique (CNRS), 75016 Paris (FR)
(72) Inventeur: PETIT, Philippe, 73310 RUFFIEUX (FR); VIALLE, Pierre-Jean, 38780 ESTRABLIN (FR); MACIUCA, Alina, 86000 POITIERS (FR); BATIOT-DUPEYRAT, Catherine, 86800 SEVRES ANXAUMONT (FR); TATI-BOUET, Jean-Michel, 86000 POITIERS (FR); ASSADI, Aymen Amin, 6080 GABES (TN); BOUZAZA, Abdelkrim, 35510 CESSON-SEVIGNE (FR); WOLBERT, Dominique, 35510 CESSON-SEVIGNE (FR); VALLET, Cédric, 38200 VIENNE (FR)
(74) Mandataire: Myon, Gérard Jean-Pierre

(56) Documents cités:
- WO-A1-2007/051912
- WO-A1-2010/130965
- WO-A2-2009/096662
- US-A1- 2003 209 501
- US-A1- 2005 118 079
- US-A1- 2007 114 465
- SANO T ET AL: "Contributions of photocatalytic/catalytic activities of TiO2 and gamma-Al2O3 in nonthermal plasma on oxidation of acetaldehyde and CO", JOURNAL OF MOLECULAR CATALYSIS A: CHEMICAL, ELSEVIER, AMSTERDAM, NL, vol. 245, no. 1-2, 15 février 2006 (2006-02-15), pages 235-241, XP028015539, ISSN: 1381-1169, DOI: 10.1016/J.MOLCATA.2005.10.002 [extrait le 2006-02-15]

## Description

La présente invention concerne un dispositif, un système et un procédé de traitement de gaz.

La photocatalyse permet d'accélérer une réaction chimique se produisant à la surface d'une substance nommée « photocatalyseur », lorsque cette dernière absorbe des photons. Généralement, le photocatalyseur est constitué de dioxyde de titane.

La photocatalyse est utilisée pour éliminer les polluants organiques présents dans l'air. Une lampe émet un rayonnement ultraviolet éclairant le photocatalyseur, qui devient alors un puissant oxydant détruisant les composés organiques volatiles (COV). Plus précisément, lorsqu'il est soumis à un rayonnement UV, le photocatalyseur libère des radicaux oxygénés à sa surface, qui dégradent les composés organiques jusqu'à ce que le carbone des chaînes carbonées soit complètement transformé en dioxyde de carbone.

WO-2009/007588 divulgue une unité de traitement de gaz par photocatalyse, comprenant un support diélectrique plat dont les deux faces comportent chacune une bande conductrice formant une électrode. Un générateur électrique alimente les électrodes, qui génèrent un plasma de surface contre le photocatalyseur. En fonctionnement, le plasma de surface constitue une source stable du rayonnement qui active le catalyseur. De cette manière, la fonction du plasma est la même que celle du rayonnement UV de la lampe. Toutefois, l'efficacité du plasma est inférieure à celle d'un rayonnement d'une lampe, de sorte que cette unité de traitement a un faible rendement.

WO-2007/051912 divulgue un dispositif de traitement d'effluents gazeux dans lequel le photocatalyseur est activé à la fois par des lampes, réparties à l'extérieur du dispositif, émettant un rayonnement ultraviolet et par un plasma froid généré par des électrodes alimentées par un générateur électrique. L'efficacité de ce dispositif de traitement est meilleure que l'unité de traitement de WO-2009/007588, dans laquelle le catalyseur est activé uniquement par un plasma froid. Par ailleurs, le photocatalyseur peut être placé perpendiculairement aux électrodes et au flux d'air, et se présente alors sous la forme d'un support perméable, par exemple en nid d'abeilles. En alternative, le photocatalyseur est disposé parallèlement aux électrodes et aux flux d'air, et se présente alors sous la forme d'une succession de couches parallèles. La fabrication d'un tel dispositif est relativement longue et coûteuse, compte tenu de l'agencement du photocatalyseur. De plus, le rayonnement ultraviolet des lampes ne permet par une irradiation optimale du photocatalyseur car les lampes sont localisées à l'extérieur du dispositif et ont une action limitée car elles éclairent localement le photocatalyseur.

C'est à ces inconvénients qu'entend plus particulièrement remédier l'invention en proposant un dispositif de traitement de gaz associant le traitement par photocatalyse et par plasma, combinant les effets des rayonnements ultraviolets sur le photocatalyseur et d'un plasma froid de surface. L'invention propose un dispositif efficace pour réduire rapidement la concentration des polluants, facile et rapide à fabriquer et ayant un coût de fabrication réduit.

A cet effet, l'invention a pour objet un dispositif de traitement de gaz, comprenant :
- un conduit diélectrique comportant une entrée et une sortie de gaz,
- une lampe générant un rayonnement au moins en partie ultraviolet, placée dans le conduit,
- une première électrode, disposée contre une paroi extérieure du conduit,
- une deuxième électrode, disposée à l'intérieur du conduit,
- un élément photocatalytique amovible, disposé contre une paroi intérieure du conduit et comprenant un support qui supporte un photocatalyseur.

La deuxième électrode est formée par un fil métallique hélicoïdal comportant des spires qui plaquent l'élément photocatalytique contre la paroi intérieure du conduit.

Grâce à l'invention, le montage de l'élément photocatalytique à l'intérieur du conduit est facile et rapide, grâce à la deuxième électrode qui plaque l'élément photocatalytique contre la paroi du conduit. Le traitement du gaz est efficace compte tenu de la géométrie de la deuxième électrode, qui permet à une grande majorité du rayonnement ultraviolet d'atteindre le photocatalyseur.

Selon des aspects avantageux mais non obligatoires de l'invention, un tel dispositif de traitement de gaz peut incorporer une ou plusieurs des caractéristiques suivantes, prises dans toute combinaison techniquement admissible :
- L'élément photocatalytique se présente sous la forme d'une feuille souple.
- La lampe est à l'intérieur d'un tube de quartz.
- La section du conduit et les spires de la deuxième électrode sont circulaires.
- Le conduit est fabriqué au moins en partie avec du verre borosilicate.
- La lampe est de forme allongée et est centrée sur un axe longitudinal du conduit.
- Le support de l'élément photocatalytique est fabriqué au moins en partie avec des fibres de verre.

L'invention concerne également un système de traitement de gaz qui comprend :
- un tel dispositif de traitement de gaz,
- un premier générateur électrique alternatif alimentant la lampe,
- un deuxième générateur électrique alternatif alimentant les électrodes,
- un organe de mise en dépression faisant circuler le gaz à traiter entre l'entrée et la sortie du conduit.

Enfin, l'invention concerne un procédé de traitement de gaz au moyen d'un tel système, qui comprend des étapes dans lesquelles :
- a) l'organe de mise en dépression fait circuler le gaz entre l'entrée et la sortie du conduit,
- b) les électrodes génèrent un plasma froid de surface contre la face avant de l'élément photocatalytique,
- c) la lampe génère un rayonnement ultraviolet vers la face avant de l'élément photocatalytique,

Avantageusement, les étapes sont simultanées.

L'invention sera mieux comprise et d'autres avantages de celle-ci apparaitront plus clairement à la lumière de la description qui va suivre d'un système, d'un dispositif et d'un procédé de traitement de gaz conformes à l'invention, donnée uniquement à titre d'exemple et faite en référence aux dessins annexés dans lesquels :
- La figure 1 est un schéma d'un système de traitement de gaz conforme à l'invention ;
- La figure 2 est une coupe schématique, à plus grande échelle, du détail II à la figure 1.

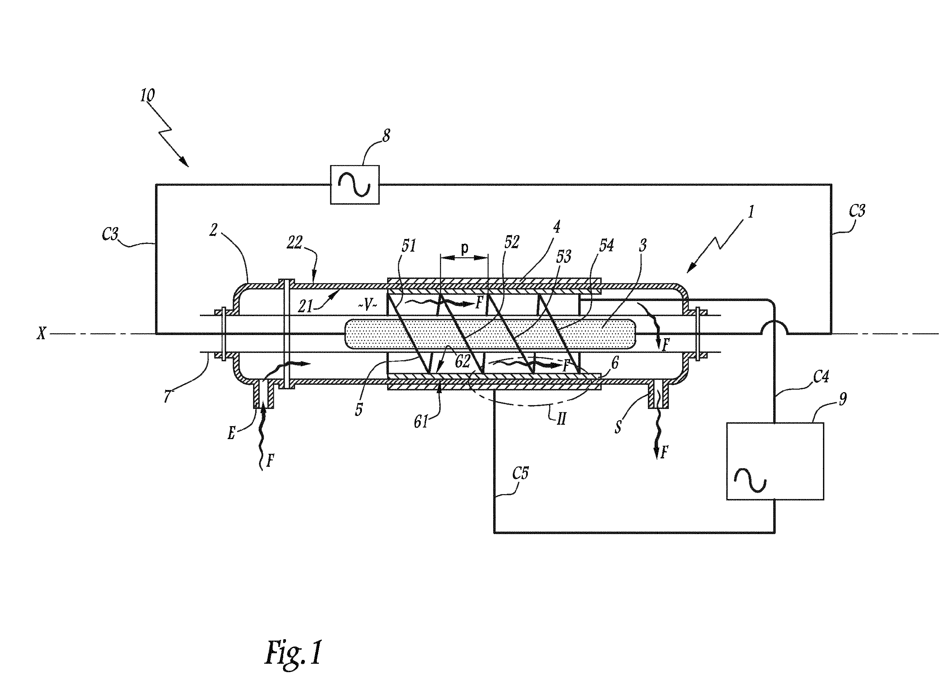

La figure 1 montre un système de traitement de gaz 10, comprenant un dispositif 1 raccordé à deux générateurs électriques alternatifs 8 et 9. Le dispositif 1 comprend un conduit 2 de forme allongée s'étendant le long d'un axe longitudinal central X et fabriqué à partir d'un matériau diélectrique, par exemple du verre borosilicate tel que le Pyrex (marque déposée). Le conduit 2 est cylindrique, à section circulaire et comporte une surface intérieure 21 et une surface extérieure 22 cylindriques, à section circulaire, situées respectivement à l'intérieur et à l'extérieur d'un volume intérieur V du conduit 2. Un organe de mise en dépression non représenté, tel qu'un ventilateur, fait circuler le gaz à traiter dans le volume V, entre une entrée E et une sortie S du conduit 2, comme représenté par les flèches F.

Une lampe 3 de forme allongée, émettant un rayonnement ultraviolet UV, est disposée dans le volume V du conduit 2. La lampe 3 est centrée sur l'axe X du conduit 2 et est alimentée par le générateur 8, qui est relié à la lampe 3 par des câbles électriques C3 s'étendant dans un tube de quartz 7 disposé autour de la lampe 3 et laissant passer le rayonnement de la lampe 3. La lampe 3 est cylindrique, à section circulaire, et son diamètre est inférieur au diamètre intérieur du conduit 2 et au diamètre du tube de quartz 7.

Le tube de quartz 7 permet de réaliser l'étanchéité du conduit 2 au niveau de ses extrémités. De plus, la connectique de la lampe 3 est protégée des réactions chimiques ayant lieu dans le conduit 2 par le tube de quartz 7. Le tube de quartz 7 laisse au moins en partie passer le rayonnement UV émis par la lampe 3.

Un élément photocatalytique 6 est plaqué contre la surface intérieure 21 du conduit 2. L'élément photocatalytique 6 se présente sous la forme d'une feuille souple comportant une face arrière 61 et une face avant 62. L'élément photocatalytique 6 comprend un support diélectrique, c'est-à-dire non conducteur, supportant un photocatalyseur réparti sur la face avant 62. Par exemple, le support est en fibres de verre et le photocatalyseur est du dioxyde de titane TiO₂.

L'élément photocatalytique 6 est enroulé, ce qui lui confère une forme cylindrique, à section circulaire. Lorsque l'élément photocatalytique 6 est installé à l'intérieur du conduit 2, sa face arrière 61 est en contact surfacique avec la surface intérieure 21 du conduit 2 et sa face avant 62 est tournée vers la lampe 3. De préférence, les faces 61 et 62 ne se chevauchent pas et les bords de l'élément photocatalytique sont affleurants, c'est-à-dire qu'ils sont en contact l'un avec l'autre. Eventuellement, les bords peuvent se chevaucher de quelques millimètres ou un espace libre peut subsister entre les bords.

Une électrode tubulaire 4 est disposée contre la surface extérieure 22 du conduit 2. Le diamètre intérieur de l'électrode 4 est égal, au jeu de fonctionnement près, au diamètre de la surface extérieure 22 du conduit 2. Ainsi, l'électrode tubulaire 4 est en contact surfacique avec la surface extérieure 22 du conduit 2.

Une électrode hélicoïdale 5 est disposée à l'intérieur du conduit 2, entre le tube de quartz 7 de la lampe 3 et l'élément photocatalytique 6. L'électrode hélicoïdale 5 est constituée par un fil enroulé pour former plusieurs spires circulaires 51, 52, 53 et 54 espacées d'un pas p. Le fil est déformable élastiquement et lorsque l'électrode hélicoïdale 5 n'est pas soumise à des efforts mécaniques, elle s'inscrit dans une enveloppe cylindrique ayant un diamètre légèrement supérieur au diamètre intérieur du conduit 2. Lorsque l'électrode hélicoïdale 5 est disposée à l'intérieur du conduit 2, elle se déforme élastiquement et son diamètre extérieur diminue, de sorte que les spires 51 à 54 plaquent radialement l'élément photocatalytique 6 contre le conduit 2, en réalisant un contact surfacique entre la face arrière 61 de l'élément photocatalytique 6 et la paroi intérieure 21 du conduit 2. Les spires 51 à 54 de l'électrode hélicoïdale 5 sont alors en contact avec la face avant 62 de l'élément photocatalytique 6, qui supporte le photocatalyseur.

Les électrodes 4 et 5 sont fabriquées à partir d'un matériau conducteur et sont alimentées par le générateur 9, par l'intermédiaire de câbles électriques C4 et C5. Par exemple, l'électrode hélicoïdale 5 est en acier inoxydable, ce qui lui permet de résister à l'oxydation générée par les réactions chimiques ayant lieu dans le conduit 2 et ce qui lui confère une souplesse et une élasticité lui permettant de plaquer l'élément photocatalytique 6 contre le conduit 2. L'électrode tubulaire 4 est par exemple en cuivre, ce qui lui permet d'être facilement plaquée contre la surface extérieure 22 du conduit 2.

En fonctionnement, un ventilateur fait circuler le gaz à traiter entre l'entrée E et la sortie S du conduit 2. Le générateur 8 alimente la lampe 3 qui génère un rayonnement ultraviolet UV sur la face avant 62 de l'élément photocatalytique 6, ce qui crée une réaction de photocatalyse.

Simultanément, le générateur 9 crée une différence de potentiel électrique entre l'électrode tubulaire 5 et l'électrode hélicoïdale 4. Un plasma froid de surface 11 se forme ainsi contre la face avant 62 de l'élément photocatalytique 6, autour des spires 51 à 54. Le plasma de surface 11 crée des vents ioniques favorisant l'homogénéisation du gaz traité, ce qui augmente l'efficacité de la photocatalyse.

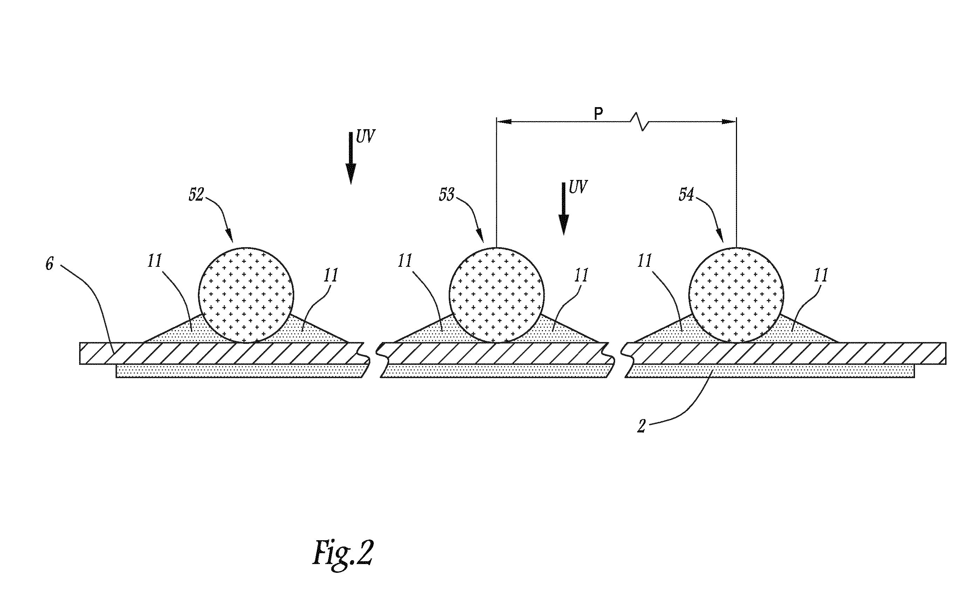

Comme visible à la figure 2, le pas p de l'électrode hélicoïdale 5 est adapté pour que le plasma de surface 11 généré autour de chaque spire 51 à 54 ne chevauche pas le plasma de surface 11 généré par une spire adjacente. De plus, le plasma de surface 11 généré par chaque spire 51 à 54 est attenant, voire sensiblement attenant, au plasma de surface 11 généré par la ou les spires adjacentes. Ainsi, la totalité, voire la quasi-totalité, de l'élément photocatalytique 6 est couvert par le plasma de surface 11.

La destruction des composés organiques présents dans le gaz à traiter est due à l'effet combiné de la photocatalyse, réalisée en activant le photocatalyseur avec le rayonnement UV, et du plasma de surface. Ceci améliore la rapidité de la réaction chimique de destruction des composés organiques présents dans le gaz.

Par rayonnement ultraviolet, on entend un rayonnement dont la longueur d'onde est comprise dans le domaine des ultraviolets, c'est-à-dire entre 100 nm et 400 nm. En fonction de la nature des polluants à éliminer, il est envisageable de sélectionner la longueur en fonction de la nature des polluants.

La lampe 3 est placée au centre du conduit 2, ce qui permet d'obtenir une puissance d'irradiation homogène du photocatalyseur. Ainsi, les effets du rayonnement ultraviolet UV sont améliorés.

Dans un autre mode de réalisation, non représenté, le conduit 2 n'est pas à section circulaire. Par exemple, le conduit 2 peut être un tube à section rectangulaire ou carrée. Dans ce cas, les spires 51 à 54 de l'électrode hélicoïdale 5 ont une forme adaptée pour plaquer l'élément photocatalytique 6 contre la paroi intérieure 21 du conduit 2. Par exemple, les spires 51 à 54 peuvent avoir la forme d'une étoile à quatre branches dont les pointes sont placées contre les arêtes de la paroi intérieure 21 du conduit 2.

Le rayonnement émis par la lampe 3 peut avoir une longueur d'onde unique, ou s'étendre sur une plage de longueurs d'onde. Dans ce cas, le rayonnement est au moins en partie ultraviolet, c'est-à-dire que ses longueurs d'ondes sont au moins en partie comprises dans le domaine des ultraviolets.

Le montage du dispositif de traitement de gaz 1 est simple et rapide : il suffit d'insérer l'électrode hélicoïdale 5 dans le conduit 2 pour réaliser le maintien en position de l'élément photocatalytique 6.

## Revendications

1. Dispositif (1) de traitement de gaz, comprenant :
- un conduit diélectrique (2) comportant une entrée (E) et une sortie (S) de gaz,
- une lampe (3) générant un rayonnement au moins en partie ultraviolet (UV), placée dans le conduit (2),
- une première électrode (4), disposée contre une paroi extérieure (22) du conduit (2),
- une deuxième électrode (5), disposée à l'intérieur du conduit (2),
- un élément photocatalytique amovible (6), disposé contre une paroi intérieure (21) du conduit (2) et comprenant un support qui supporte un photocatalyseur,
**caractérisé en ce que** la deuxième électrode (5) est formée par un fil métallique hélicoïdal comportant des spires (51, 52, 53, 54) qui plaquent l'élément photocatalytique (6) contre la paroi intérieure (21) du conduit (2).

2. Dispositif (1) selon la revendication 1, **caractérisé en ce que** l'élément photocatalytique (6) se présente sous la forme d'une feuille souple.

3. Dispositif (1) selon l'une des revendications précédentes, **caractérisé en ce que** la lampe (3) est à l'intérieur d'un tube de quartz (7).

4. Dispositif (1) selon l'une des revendications précédentes, **caractérisé en ce que** la section du conduit (2) et les spires (51-54) de la deuxième électrode (5) sont circulaires.

5. Dispositif (1) selon l'une des revendications précédentes, **caractérisé en ce que** le conduit (2) est fabriqué au moins en partie avec du verre borosilicate.

6. Dispositif (1) selon l'une des revendications précédentes, **caractérisé en ce que** la lampe (3) est de forme allongée et est centrée sur un axe longitudinal (X) du conduit (2).

7. Dispositif (1) selon l'une des revendications précédentes, **caractérisé en ce que** le support de l'élément photocatalytique (6) est fabriqué au moins en partie avec des fibres de verre.

8. Système (10) de traitement de gaz, **caractérisé en ce qu'**il comprend :
- un dispositif (1) selon l'une des revendications précédentes,
- un premier générateur électrique alternatif (8) alimentant la lampe (3),
- un deuxième générateur électrique alternatif (9) alimentant les électrodes (4, 5),
- un organe de mise en dépression faisant circuler le gaz à traiter entre l'entrée (E) et la sortie (S) du conduit (2).

9. Procédé de traitement de gaz au moyen d'un système (10) selon la revendication 8, **caractérisé en ce qu'**il comprend des étapes dans lesquelles :
- a) l'organe de mise en dépression fait circuler le gaz entre l'entrée (E) et la sortie (S) du conduit (2),
- b) les électrodes (4, 5) génèrent un plasma froid de surface (11) contre la face avant (62) de l'élément photocatalytique (6),
- c) la lampe (3) génère un rayonnement ultraviolet (UV) vers la face avant (62) de l'élément photocatalytique (6).

10. Procédé selon la revendication 9, **caractérisé en ce que** les étapes sont simultanées.

## Patentansprüche

1. Vorrichtung (1) zur Gasbehandlung,
die Folgendes aufweist :
- eine dielektrische Leitungsröhre (2), die einen Eingang (E) und einen Ausgang (S) für Gas aufweist,
- eine in der Leitungsröhre (2) angeordnete Lampe (3), die eine zumindest zum Teil ultraviolette (UV) Strahlung erzeugt,
- eine erste Elektrode (4), die an einer Außenwand (22) der Leitungsröhre (2) angeordnet ist,
- ein entfernbares photokatalytisches Element (6), das an einer Innenwand (21) der Leitungsröhre (2) angeordnet ist und einen Träger aufweist, der einen Photokatalysator trägt,
**dadurch gekennzeichnet,**
**dass** die zweite Elektrode (5) aus einem wendelförmigen Metallfaden gebildet ist, der Windungen (51, 52, 53, 54) aufweist, die das photokatalytische Element (6) gegen die Innenwand (21) der Leitungsröhre (2) drücken.

2. Vorrichtung (1) nach Anspruch 1,
**dadurch gekennzeichnet,**
**dass** das photokatalytische Element (6) in der Form eines biegsamen Flächenelements vorliegt.

3. Vorrichtung (1) nach einem der vorhergehenden Ansprüche,
**dadurch gekennzeichnet,**
**dass** die Lampe (3) im Inneren eines Quartzrohres (7) angeordnet ist.

4. Vorrichtung (1) nach einem der vorhergehenden Ansprüche,
**dadurch gekennzeichnet,**
**dass** der Querschnitt der Leitungsröhre und die Windungen (51-54) der zweiten Elektrode (5) kreisförmig ausgebildet sind.

5. Vorrichtung (1) nach einem der vorhergehenden Ansprüche,
**dadurch gekennzeichnet,**
**dass** die Leitungsröhre (2) zumindest teilweise mit Borosilicatglas hergestellt ist.

6. Vorrichtung (1) nach einem der vorhergehenden Ansprüche,
**dadurch gekennzeichnet,**
**dass** die Lampe (3) eine langgestreckte Form aufweist und längs einer Längsachse (X) der Leitungsröhre (2) zentriert ist.

7. Vorrichtung (1) nach einem der vorhergehenden Ansprüche,
**dadurch gekennzeichnet,**
**dass** der Träger des photokatalytischen Elements (6) zumindest zum Teil mit Glasfasern hergestellt ist.

8. System (10) zur Behandlung von Gas,
**dadurch gekennzeichnet,**
**dass** es folgendes aufweist:
- eine Vorrichtung (1) nach einem der vorhergehenden Ansprüche,
- einen ersten Wechselstromgenerator (8), der die Lampe (3) versorgt,
- einen zweiten Wechselstromgenerator (9), der die Elektroden (4, 5) versorgt, und
- ein Unterdruck-Erzeugungsorgan, welches das zu behandelnde Gas zwischen dem Eingang (E) und dem Ausgang (S) der Leitungsröhre (2) zirkulieren lässt.

9. Verfahren zur Behandlung von Gas mit einem System (10) nach Anspruch 8,
**dadurch gekennzeichnet,**
**dass** es Schritte aufweist, bei denen:
- a) das Unterdruck-Erzeugungsorgan das Gas zwischen dem Eingang (E) und dem Ausgang (S) der Leitungsröhre (2) zirkulieren lässt,
- b) die Elektroden (4, 5) ein kaltes Oberflächenplasma (11) an der Vorderseite (62) des photokatalytischen Elements (6) erzeugen, und
- c) die Lampe (3) eine ultraviolette (UV) Strahlung in Richtung der Vorderseite (62) des photokatalytischen Elements (6) erzeugt.

10. Verfahren nach Anspruch 9,
**dadurch gekennzeichnet,**
**dass** die Schritte gleichzeitig durchgeführt werden.

## Claims

1. Device (1) for gas treatment, comprising:
- a dielectric conduit (2) comprising an inlet (E) and an outlet (S) for gas,
- a lamp (3) generating at least partly ultraviolet radiation (UV), placed in the conduit (2),
- a first electrode (4), disposed against an exterior wall (22) of the conduit (2),
- a second electrode (5) disposed inside the conduit (2),
- a moveable photocatalytic element (6), disposed against an interior wall (21) of the conduit (2) and comprising a support which supports a photocatalyst,
**characterised in that** the second electrode (5) is formed by a helicoidal metallic wire comprising turns (51, 52, 53, 54) which place the photocatalytic element (6) against the interior wall (21) of the conduit (2).

2. Device (1) according to claim 1, **characterised in that** the photocatalytic element (6) is in the form of a flexible foil.

3. Device (1) according to one of the preceding claims, **characterised in that** the lamp (3) is inside a quartz tube (7).

4. Device (1) according to one of the preceding claims, **characterised in that** the section of the conduit (2) and the turns (51 - 54) of the second electrode (5) are circular.

5. Device (1) according to one of the preceding claims, **characterised in that** the conduit (2) is manufactured at least in part with borosilicate glass.

6. Device (1) according to one of the preceding claims, **characterised in that** the lamp (3) is of an elongated form and is centred on a longitudinal axis (X) of the conduit (2).

7. Device (1) according to one of the preceding claims, **characterised in that** the support of the photocatalytic element (6) is manufactured at least in part with glass fibres.

8. System (10) for gas treatment, **characterised in that** it comprises:
- a device (1) according to one of the preceding claims,
- a first alternating electrical generator (8) feeding the lamp (3),
- a second alternating electrical generator (9) feeding the electrodes (4, 5),
- an element for lowering pressure which circulates the gas to be treated between the inlet (E) and the outlet (S) of the conduit (2).

9. Method for gas treatment by means of a system (10) according to claim 8, **characterised in that** it comprises steps in which:
a) the element for lowering the pressure circulates the gas between the inlet (E) and outlet (S) of the conduit (2),
b) the electrodes (4, 5) generate a cold surface plasma (11) against the front face (62) of the photocatalytic element (6),
c) the lamp (3) generates ultraviolet radiation (UV) towards the front face (62) of the photocatalytic element (6).

10. Method according to claim 9, **characterised in that** the steps are simultaneous.
